# EUROPEAN PATENT APPLICATION

(11) **EP 4 014 974 A1**
(43) Date of publication of application: **22.06.2022**
(21) Application number: 20215208.8
(22) Date of filing: 17.12.2020
(51) Int. Cl.: A61K 31/355, A61K 31/4172, A61P 15/08, A61K 9/00, A61K 9/06

(54) **A COMPOSITION FOR USE IN THE TREATMENT OF INFERTILITY**

(71) Applicant: The Provost, Fellows, Foundation Scholars, and The Other Members of Board, of The College of The Holy and Undivided Trinity of Queen Elizabeth, Dublin 2 (IE)
(72) Inventor: KELLY, Vincent, Dublin, 2 (IE); BROWNING, Jill, Dublin, 2 (IE); JAGOE, William, Dublin, 2 (IE); HEALY, Anne Marie, Dublin, 2 (IE); FITZPATRICK, Anna, Dublin, 2 (IE); lHENRIQUES DO AMARAL, Lilian, Dublin, 2 (IE)
(74) Representative: FRKelly

(57) **Abstract**

The present invention relates to a composition for use in the treatment of infertility. The composition of the invention finds utility as a personal lubricant that can be used to increase sperm motility or sperm velocity. Also disclosed are methods of treating infertility.

## Description

### Field of the Invention

The present invention relates to a composition for use in the treatment of infertility. The composition of the invention finds utility as a personal lubricant that can be used to increase sperm motility or sperm velocity.

### Background to the Invention

In the western world, approximately 1 in 6 couples experience problems with infertility. The male partner contributes to over 50% of such cases. This is largely due to DNA damage of sperm cells or problems arising from mitochondrial function resulting from the damaging effects of reactive oxygen species (ROS).

Many of the current vaginal lubricants marketed for aiding couples attempting to conceive have been shown to be toxic to sperm and to reduce either motility or forward progression of sperm.

An eminent need exists for a lubricant composition that can maintain sperm motility and progression, in addition to protecting sperm DNA integrity. In the *in utero* context, the lubricant composition should show no adverse effects on the sperm, the egg, embryo fertilisation and development or on the integrity of the female reproductive environment.

### Summary of the Invention

According to a first aspect of the present invention, there is provided a composition comprising an antioxidant for use in the treatment of infertility.

According to a second aspect of the present invention, there is provided use of a composition comprising an antioxidant in the manufacture of a medicament for use in the treatment of infertility.

According to a third aspect of the present invention, there is provided a method for the treatment of infertility, the method comprising the step of administering a composition comprising an antioxidant to a subject.

Optionally, the antioxidant is a derivative of an amino acid. Further optionally, the antioxidant is a derivative of histidine. Still further optionally, the antioxidant is a thiourea derivative of histidine. Still further optionally, the antioxidant is ergothionine.

Preferably, the antioxidant is ergothionine.

Optionally, the composition comprises 0.1 - 20.0mM; optionally 0.1 - 17.5mM; optionally 0.1 - 15.0mM; optionally 0.1 - 12.5mM; optionally 0.1 - 11.5mM; optionally 0.1 - 10mM; optionally 0.1 - 7.5mM; optionally 0.1 - 5mM; optionally 0.1 - 2.5mM; optionally 0.1 - 1 mM; optionally 0.25 - 1 mM; optionally 0.5 - 1 mM; optionally 0.75 - 1 mM; optionally 1 mM antioxidant.

Optionally, the composition comprises 0.1mM, 0.25mM, 0.5mM, 1.0mM, 2.5mM, 5mM, 7.5mM, 10mM, 11.5mM, 12.5mM, 15.0mM 17.5mM, or 20.0mM antioxidant.

Preferably, the composition comprises 1mM antioxidant.

Optionally, the composition comprises 0.1 - 20.0mM; optionally 0.1 - 17.5mM; optionally 0.1 - 15.0mM; optionally 0.1 - 12.5mM; optionally 0.1 - 11.5mM; optionally 0.1 - 10mM; optionally 0.1 - 7.5mM; optionally 0.1 - 5mM; optionally 0.1 - 2.5mM; optionally 0.1 - 1mM; optionally 0.25 - 1mM; optionally 0.5 - 1 mM; optionally 0.75 - 1 mM; optionally 1 mM derivative of an amino acid.

Optionally, the composition comprises 0.1mM, 0.25mM, 0.5mM, 1.0mM, 2.5mM, 5mM, 7.5mM, 10mM, 11.5mM, 12.5mM, 15.0mM 17.5mM, or 20.0mM derivative of an amino acid.

Preferably, the composition comprises 1mM derivative of an amino acid.

Optionally, the composition comprises 0.1 - 20.0mM; optionally 0.1 - 17.5mM; optionally 0.1 - 15.0mM; optionally 0.1 - 12.5mM; optionally 0.1 - 11.5mM; optionally 0.1 - 10mM; optionally 0.1 - 7.5mM; optionally 0.1 - 5mM; optionally 0.1 - 2.5mM; optionally 0.1 - 1mM; optionally 0.25 - 1mM; optionally 0.5 - 1 mM; optionally 0.75 - 1 mM; optionally 1 mM derivative of histidine.

Optionally, the composition comprises 0.1mM, 0.25mM, 0.5mM, 1.0mM, 2.5mM, 5mM, 7.5mM, 10mM, 11.5mM, 12.5mM, 15.0mM 17.5mM, or 20.0mM derivative of histidine.

Preferably, the composition comprises 1mM derivative of histidine.

Optionally, the composition comprises 0.1 - 20.0mM; optionally 0.1 - 17.5mM; optionally 0.1 - 15.0mM; optionally 0.1 - 12.5mM; optionally 0.1 - 11.5mM; optionally 0.1 - 10mM; optionally 0.1 - 7.5mM; optionally 0.1 - 5mM; optionally 0.1 - 2.5mM; optionally 0.1 - 1mM; optionally 0.25 - 1mM; optionally 0.5 - 1 mM; optionally 0.75 - 1 mM; optionally 1 mM thiourea derivative of histidine.

Optionally, the composition comprises 0.1mM, 0.25mM, 0.5mM, 1.0mM, 2.5mM, 5mM, 7.5mM, 10mM, 11.5mM, 12.5mM, 15.0mM 17.5mM, or 20.0mM thiourea derivative of histidine.

Preferably, the composition comprises 1mM thiourea derivative of histidine.

Optionally, the composition comprises 0.1 - 20.0mM; optionally 0.1 - 17.5mM; optionally 0.1 - 15.0mM; optionally 0.1 - 12.5mM; optionally 0.1 - 11.5mM; optionally 0.1 - 10mM; optionally 0.1 - 7.5mM; optionally 0.1 - 5mM; optionally 0.1 - 2.5mM; optionally 0.1 - 1 mM; optionally 0.25 - 1 mM; optionally 0.5 - 1 mM; optionally 0.75 - 1 mM; optionally 1 mM ergothionine.

Optionally, the composition comprises 0.1 mM, 0.25mM, 0.5mM, 1.0mM, 2.5mM, 5mM, 7.5mM, 10mM, 11.5mM, 12.5mM, 15.0mM 17.5mM, or 20.0mM ergothionine.

Preferably, the composition comprises 1mM ergothionine.

Optionally, the composition further comprises an excipient.

Optionally, the excipient is a viscoelastic polymer.

Optionally, the excipient is cellulose. Further optionally, the excipient is methylcellulose. Still further optionally, the excipient is propyl methylcellulose. Still further optionally, the excipient is hydroxypropyl methylcellulose (HPMC).

Preferably, the excipient is HPMC.

Optionally, the composition comprises 0.8 - 1.6 wt%, optionally 1.0 - 1.6 wt%, optionally 1.0 - 1.4 wt%, optionally 1.0 - 1.2 wt% excipient.

Optionally, the composition comprises 0.8, 1.0, 1.2, 1.4 or 1.6 wt% excipient.

Preferably, the composition comprises 1.2 wt% excipient.

Optionally, the composition comprises 0.8 - 1.6 wt%, optionally 1.0 - 1.6 wt%, optionally 1.0 - 1.4 wt%, optionally 1.0 - 1.2 wt% viscoelastic polymer.

Optionally, the composition comprises 0.8, 1.0, 1.2, 1.4 or 1.6 wt% viscoelastic polymer.

Preferably, the composition comprises 1.2 wt% viscoelastic polymer.

Optionally, the composition comprises 0.8 - 1.6 wt%, optionally 1.0 - 1.6 wt%, optionally 1.0 - 1.4 wt%, optionally 1.0 - 1.2 wt% cellulose.

Optionally, the composition comprises 0.8, 1.0, 1.2, 1.4 or 1.6 wt% cellulose.

Preferably, the composition comprises 1.2 wt% cellulose.

Optionally, the composition comprises 0.8 - 1.6 wt%, optionally 1.0 - 1.6 wt%, optionally 1.0 - 1.4 wt%, optionally 1.0 - 1.2 wt% methylcellulose.

Optionally, the composition comprises 0.8, 1.0, 1.2, 1.4 or 1.6 wt% methylcellulose.

Preferably, the composition comprises 1.2 wt% methylcellulose.

Optionally, the composition comprises 0.8 - 1.6 wt%, optionally 1.0 - 1.6 wt%, optionally 1.0 - 1.4 wt%, optionally 1.0 - 1.2 wt% propyl methylcellulose.

Optionally, the composition comprises 0.8, 1.0, 1.2, 1.4 or 1.6 wt% propyl methylcellulose.

Preferably, the composition comprises 1.2 wt% propyl methylcellulose.

Optionally, the composition comprises 0.8 - 1.6 wt%, optionally 1.0 - 1.6 wt%, optionally 1.0 - 1.4 wt%, optionally 1.0 - 1.2 wt% HPMC.

Optionally, the composition comprises 0.8, 1.0, 1.2, 1.4 or 1.6 wt% HPMC.

Preferably, the composition comprises 1.2 wt% HPMC.

Optionally, the composition further comprises a thickening agent.

Optionally, the thickening agent is a carboxylic acid. Further optionally, the thickening agent is an unsaturated carboxylic acid. Still further optionally, the thickening agent is an acrylic acid. Still further optionally, the thickening agent is a polyacrylic acid. Still further optionally, the thickening agent is a synthetic polyacrylic acid. Still further optionally, the thickening agent is carboxypolymethylene.

Optionally, the thickening agent is a crosslinked polyacrylic acid. Further optionally, the thickening agent is a polyacrylic acid crosslinked with allyl sucrose. Alternatively, the thickening agent is a polyacrylic acid crosslinked with allyl pentaerythritol.

Optionally, the thickening agent further comprises a block copolymer of polyethylene glycol and a long chain alkyl acid ester. Alternatively, the thickening agent is a crosslinked polyacrylic acid further comprising a block copolymer of polyethylene glycol and a long chain alkyl acid ester. Further alternatively, the thickening agent is a polyacrylic acid crosslinked with allyl sucrose further comprising a block copolymer of polyethylene glycol and a long chain alkyl acid ester. Still further alternatively, the thickening agent is a polyacrylic acid crosslinked with allyl pentaerythritol further comprising a block copolymer of polyethylene glycol and a long chain alkyl acid ester. Still further alternatively, the thickening agent is a polyacrylic acid and C10-C30 alkyl acrylate crosslinked with allyl pentaerythritol further comprising a block copolymer of polyethylene glycol and a long chain alkyl acid ester.

Preferably, the composition comprises a polyacrylic acid.

Optionally, the composition comprises 0.1 - 1.0 wt%, optionally 0.1 - 0.8 wt%, optionally 0.1 - 0.6 wt%, optionally 0.2 - 0.6 wt% thickening agent.

Optionally, the composition comprises 0.1 wt%, 0.2 wt%, 0.3 wt%, 0.4 wt%, 0.5 wt%, 0.6 wt%, 0.7 wt%, 0.8 wt%, 0.9 wt%, 1.0 wt% thickening agent.

Preferably, the composition comprises 0.4 wt% thickening agent.

Optionally, the composition comprises 0.1 - 1.0 wt%, optionally 0.1 - 0.8 wt%, optionally 0.1 - 0.6 wt%, optionally 0.2 - 0.6 wt% carboxylic acid.

Optionally, the composition comprises 0.1 wt%, 0.2 wt%, 0.3 wt%, 0.4 wt%, 0.5 wt%, 0.6 wt%, 0.7 wt%, 0.8 wt%, 0.9 wt%, 1.0 wt% carboxylic acid.

Preferably, the composition comprises 0.4 wt% carboxylic acid.

Optionally, the composition comprises 0.1 - 1.0 wt%, optionally 0.1 - 0.8 wt%, optionally 0.1 - 0.6 wt%, optionally 0.2 - 0.6 wt% unsaturated carboxylic acid.

Optionally, the composition comprises 0.1 wt%, 0.2 wt%, 0.3 wt%, 0.4 wt%, 0.5 wt%, 0.6 wt%, 0.7 wt%, 0.8 wt%, 0.9 wt%, 1.0 wt% unsaturated carboxylic acid.

Preferably, the composition comprises 0.4 wt% unsaturated carboxylic acid.

Optionally, the composition comprises 0.1 - 1.0 wt%, optionally 0.1 - 0.8 wt%, optionally 0.1 - 0.6 wt%, optionally 0.2 - 0.6 wt% acrylic acid.

Optionally, the composition comprises 0.1 wt%, 0.2 wt%, 0.3 wt%, 0.4 wt%, 0.5 wt%, 0.6 wt%, 0.7 wt%, 0.8 wt%, 0.9 wt%, 1.0 wt% acrylic acid.

Preferably, the composition comprises 0.4 wt% acrylic acid.

Optionally, the composition comprises 0.1 - 1.0 wt%, optionally 0.1 - 0.8 wt%, optionally 0.1 - 0.6 wt%, optionally 0.2 - 0.6 wt% polyacrylic acid.

Optionally, the composition comprises 0.1 wt%, 0.2 wt%, 0.3 wt%, 0.4 wt%, 0.5 wt%, 0.6 wt%, 0.7 wt%, 0.8 wt%, 0.9 wt%, 1.0 wt% polyacrylic acid.

Preferably, the composition comprises 0.4 wt% polyacrylic acid.

Optionally, the composition comprises 0.1 - 1.0 wt%, optionally 0.1 - 0.8 wt%, optionally 0.1 - 0.6 wt%, optionally 0.2 - 0.6 wt% synthetic polyacrylic acid.

Optionally, the composition comprises 0.1 wt%, 0.2 wt%, 0.3 wt%, 0.4 wt%, 0.5 wt%, 0.6 wt%, 0.7 wt%, 0.8 wt%, 0.9 wt%, or 1.0 wt% synthetic polyacrylic acid.

Preferably, the composition comprises 0.4 wt% synthetic polyacrylic acid.

Optionally, the composition further comprises at least one salt. Further optionally, the composition further comprises at least two salts. Still further optionally, the composition further comprises two salts.

Optionally, the composition further comprises at least one sodium salt. Further optionally, the composition further comprises at least two sodium salts. Still further optionally, the composition further comprises two sodium salts.

Optionally, the composition further comprises at least one salt, the or each salt independently selected from NaCl and NaPO₄. Further optionally, the composition further comprises at least two salts, each salt independently selected from NaCl and NaPO₄. Still further optionally, the composition further comprises two salts, each salt independently selected from NaCl and NaPO₄.

Optionally, the composition comprises 2.5 - 7.5 mM salt.

Optionally, the composition comprises 2.5, 5.0, or 7.5 mM salt.

Preferably, the composition comprises 5.0 mM salt.

Optionally, the composition comprises 2.5 - 7.5 mM salt of at least one salt selected from NaCl and NaPO₄. Further optionally, the composition comprises 2.5, 5.0, or 7.5 mM salt of at least one salt selected from NaCl and NaPO₄.

Preferably, the composition comprises 5.0 mM salt of at least one salt selected from NaCl and NaPO₄.

Optionally, the composition comprises an antioxidant, an excipient, a thickening agent and at least one salt.

Further optionally, the composition comprises an amino acid, a viscoelastic polymer, a carboxylic acid and at least two salts.

Still further optionally, the composition comprises a derivative of histidine, a cellulose, an acrylic acid and two salts.

Still further optionally, the composition comprises a thiourea derivative of histidine, a methylcellulose, a polyacrylic acid and at least one salt, each independently selected from NaCl and NaPO₄.

Still further optionally, the composition comprises ergothionine, a propylmethylcellulose, a synthetic polyacrylic acid and at least two salts, each independently selected from NaCl and NaPO₄.

Preferably, the composition comprises ergothionine, HPMC, a synthetic polyacrylic acid and two salts, each independently selected from NaCl and NaPO₄.

Optionally, the composition comprises 0.1 - 20mM ergothionine, 0.8 - 1.6 wt% HPMC, 0.1 - 1.0 wt% synthetic polyacrylic acid and 2.5 - 7.5 mM of two salts, each independently selected from NaCl and NaPO₄.

Further optionally, the composition comprises 0.1mM, 0.25mM, 0.5mM, 1.0mM, 2.5mM, 5mM, 7.5mM, 10mM, 11.5mM, 12.5mM, 15.0mM 17.5mM, or 20.0mM ergothionine, 0.8, 1.0, 1.2, 1.4 or 1.6 wt% HPMC, 0.1 wt%, 0.2 wt%, 0.3 wt%, 0.4 wt%, 0.5 wt%, 0.6 wt%, 0.7 wt%, 0.8 wt%, 0.9 wt%, or 1.0 wt% synthetic polyacrylic acid and 2.5, 5.0, or 7.5 mM salt of two salts selected from NaCl and NaPO₄.

Preferably, the composition comprises 1.0mM ergothionine, 1.2 wt% HPMC, 0.4 wt% synthetic polyacrylic acid and 5.0 mM salt of two salts selected from NaCl and NaPO₄.

Optionally, the composition further comprises at least one preservative.

Optionally, the composition comprises at least 0.1 %(w/w) preservative. Further optionally, the composition comprises at least 0.25%(w/w), optionally 0.5%(w/w), optionally at least 0.75%(w/w), optionally at least 1%(w/w), optionally at least 1.25%(w/w), optionally at least 1.5%(w/w), optionally at least 1.75%(w/w) preservative.

Optionally, the at least one preservative is phenethyl alcohol (2-Phenylethan-1-ol). Further optionally, the at least one preservative comprises greater than 50%(w/w) phenethyl alcohol. Still further optionally, the at least one preservative comprises greater than 55%(w/w), optionally greater than 60%(w/w), optionally greater than 65%(w/w), optionally greater than 70%(w/w) phenethyl alcohol.

Optionally or additionally, the at least one preservative is caprylyl glycol (octane-1,2-diol). Further optionally, the at least one preservative comprises up to 50%(w/w) caprylyl glycol. Still further optionally, the at least one preservative comprises up to 45%(w/w), optionally up to 40%(w/w), optionally up to 35%(w/w), optionally up to 30%(w/w) caprylyl glycol.

Optionally, the at least one preservative is selected from phenethyl alcohol and caprylyl glycol. Further optionally, the at least one preservative is phenethyl alcohol (2-Phenylethan-1-ol) and caprylyl glycol. Still further optionally, the at least one preservative comprises greater than 50%(w/w) phenethyl alcohol and up to50%(w/w) caprylyl glycol.

Optionally, the composition has an osmolarity of 260 - 360 mOsm/kg. Further optionally, the composition has an osmolarity of 260, 270, 280, 290, 300, 310, 320, 330, 340, 350, or 360 mOsm/kg. Still further optionally, the composition has an osmolarity of 310 mOsm/kg.

Preferably, the composition has an osmolarity of 310 mOsm/kg.

Optionally, the composition has a pH of 7.0 - 8.5. Further optionally, the composition has a pH of 7.0, 7.1, 7.2, 7.3, 7.4, 7.5, 7.6, 7.7, 7.8, 7.9, 8.0, 8.1, 8.2, 8.3, 8.4, or 8.5.

Optionally, the composition has a pH between 7.0 - 7.2. Further optionally, the composition has a pH of 7.0, 7.1, or 7.2.

Preferably, the composition has a pH of 7.1.

Optionally, the composition has a viscosity of less than 2500, optionally less than 2000, optionally less than 1500, optionally less than 1000, optionally less than 500, optionally less than 250, optionally less than 100, optionally less than 50 Pa.s at 20°C.

Optionally, the composition has a viscosity of 3-30, optionally 6-20, optionally 8-18, optionally 10-16, optionally 12-14 Pa.s at 20°C. Further optionally, the composition has a viscosity of 3, 6, 8, 10, 12, 14, 16, 18, 20, or 30 Pa.s at 20°C.

Optionally, the composition is water-soluble.

Optionally, the use or the method comprises administering the composition to a subject.

Optionally, the use or the method comprises topically administering the composition to a subject.

Optionally, the subject is a member of the phylum chordata. Further optionally, the subject is a member of the superclass tetrapoda. Still further optionally, the subject is a member of the class: synapsida (mammals), sauropsida (reptiles or birds), or amphibia (amphibians).

Preferably, the subject is a human subject.

Optionally, the subject is a male subject. Preferably, the subject is a male human subject.

Optionally, the composition is a pharmaceutical composition.

Optionally, the composition is a pharmaceutical composition comprising an antioxidant and an excipient.

Further optionally, the composition is a pharmaceutical composition comprising an antioxidant and a pharmaceutically acceptable excipient.

Optionally, the excipient is a viscoelastic polymer.

Optionally, the excipient is cellulose. Further optionally, the excipient is methylcellulose. Still further optionally, the excipient is propyl methylcellulose. Still further optionally, the excipient is hydroxypropyl methylcellulose (HPMC).

Preferably, the excipient is HPMC.

Optionally, the pharmaceutical composition comprises 0.8 - 1.6 wt%, optionally 1.0 - 1.6 wt%, optionally 1.0 - 1.4 wt%, optionally 1.0 - 1.2 wt% excipient.

Optionally, the pharmaceutical composition comprises 0.8, 1.0, 1.2, 1.4 or 1.6 wt% excipient.

Preferably, the pharmaceutical composition comprises 1.2 wt% excipient.

Optionally, the composition is for use in maintaining fertility. Further optionally, the composition is for use in increasing fertility.

Optionally, the composition is for use in maintaining sperm motility. Further optionally, the composition is for use in increasing sperm motility.

Optionally, the composition is for use in maintaining sperm velocity. Further optionally, the composition is for use in increasing sperm velocity.

According to a fourth aspect of the present invention, there is provided a method for the manufacture of a composition for use in the treatment of infertility, the method comprising the step of providing a composition comprising an antioxidant.

Optionally, the method comprises the step of providing a pharmaceutical composition comprising an antioxidant and an excipient.

Optionally, the method comprises the step of providing a pharmaceutical composition comprising an antioxidant and a pharmaceutically acceptable excipient.

Optionally, the method comprises the step of providing a pharmaceutical composition by admixing an antioxidant and an excipient.

Optionally, the method comprises the step of providing a pharmaceutical composition by admixing an antioxidant and a pharmaceutically acceptable excipient.

Optionally, the composition is a lubricant. Further optionally, the composition is a personal lubricant.

### Brief Description of the Drawings

Embodiments of the present invention will be described with reference to the appended non-limiting examples and the accompanying drawings in which:

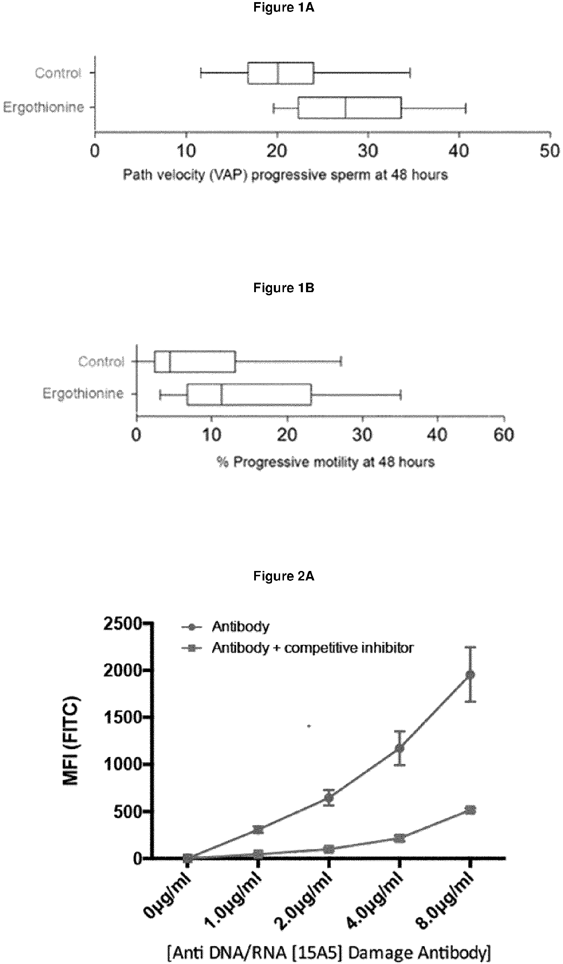

**Figure 1** illustrates the evaluation of the cytoprotective and antioxidant compound ergothionine for ability to preserve sperm motility and progression, wherein sperm were separated from seminal fluid by density gradient centrifugation, washed in sperm wash media, and incubated in multipurpose handling medium for 48 hours in the presence of antioxidant, wherein path velocity (A) and sperm motility (B) were quantified by CASA, wherein for statistical analysis the log ratio to control was used as the dependent variable in ONEWAY ANOVA with posthoc testing by Dunnet's test (comparisons against a single 'control' value only), wherein N>12 per group;

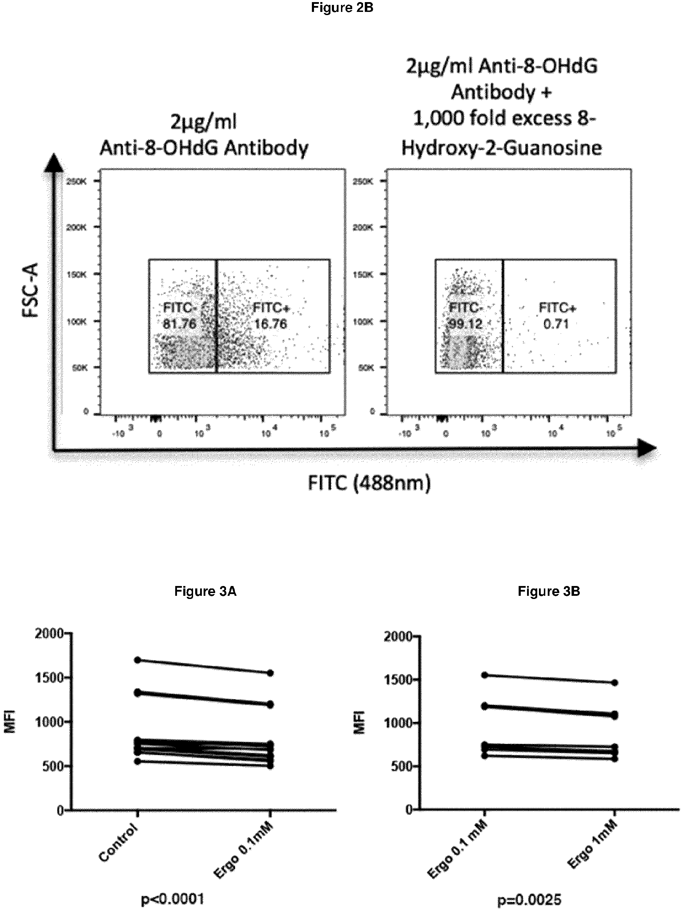

**Figure 2** illustrates optimisation of a flow cytometric assay to detect 8-OHG on sperm DNA, wherein sperm were separated from seminal fluid by washing twice in sperm wash media and fixed in 4% PFA, stained with FITC labelled 8-OHG antibody (2 µg/ml) in 0.1% Triton-X in 0.1% Sodium Citrate for 60 mins, washed, and analysed by flow cytometry, wherein data were analyzed using FlowJo software and the mean fluorescent intensity (MFI) plotted, wherein a concentration/competition assay (asterix marks final concentration) (A) and flow cytometry dot plots show competitive inhibition in the presence of 8-hydroxy-2-guanosine (B), which was quantified and analysed using FlowJo and Prism, wherein error bars ±SD, and N=2 per group (n=3 technical replicates);
**Figure 3** illustrates evaluating the effect of ergothionine on sperm oxidative DNA damage, wherein sperm were separated from seminal fluid by density gradient centrifugation, washed in sperm wash media, and incubated in multipurpose handling medium for 48 hours in the presence of the cytoprotective and antioxidant compound ergothionine, wherein sperm were fixed in 4% PFA and stained with FITC labelled 8-OHG antibody (2µg/ml) for 60 mins, washed and analysed by flow cytometry, wherein data were analyzed using FlowJo software and the mean fluorescent intensity (MFI) plotted, wherein statistical analysis was performed using the Wilcoxon signed-rank test, wherein N=6 per group;

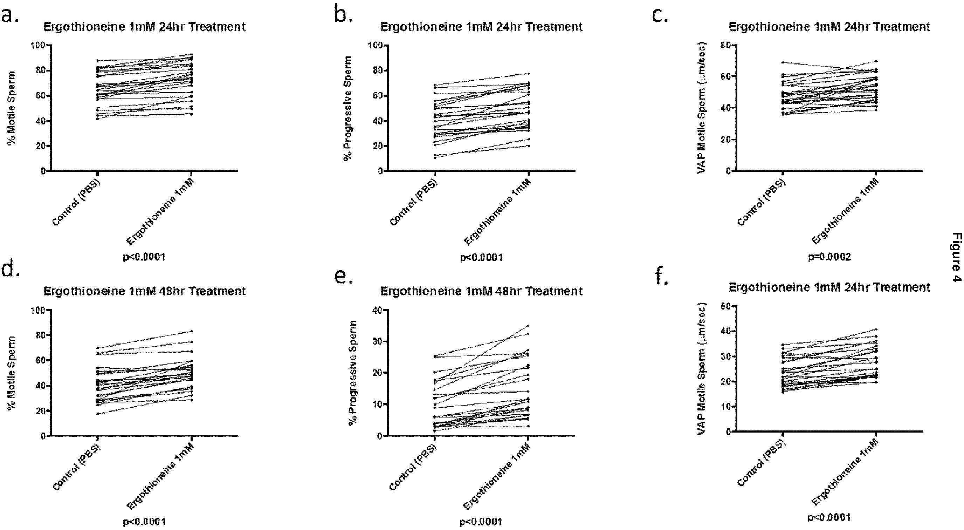

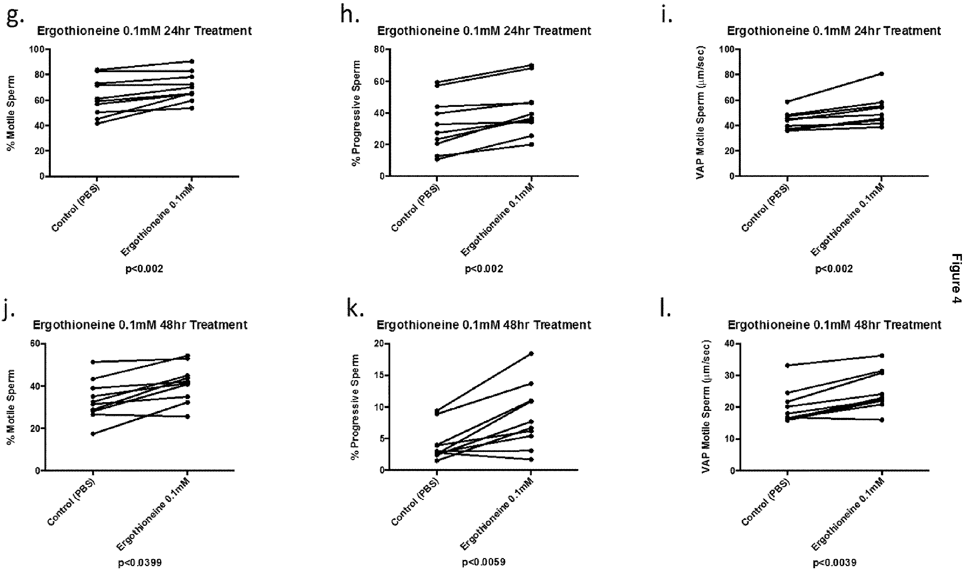

**Figure 4** illustrates ergothionine effectively maintains sperm motility and progression, wherein sperm were separated from seminal fluid by density gradient centrifugation, washed in sperm wash media and incubated in multipurpose handling medium in the presence of ergothionine at 1 mM for 24 hours (A-C) or 48 hours (D-F) or in the presence of ergothionine at 0.1 mM for 24 hours (G-I) or 48 hours (J-L),wherein sperm motility was analysed using CASA, wherein statistical analysis was performed using the Wilcoxon signed-rank, wherein N=26 per group for ergothionine at 1 mM and N=10 per group for 0.1 mM ergothionine;

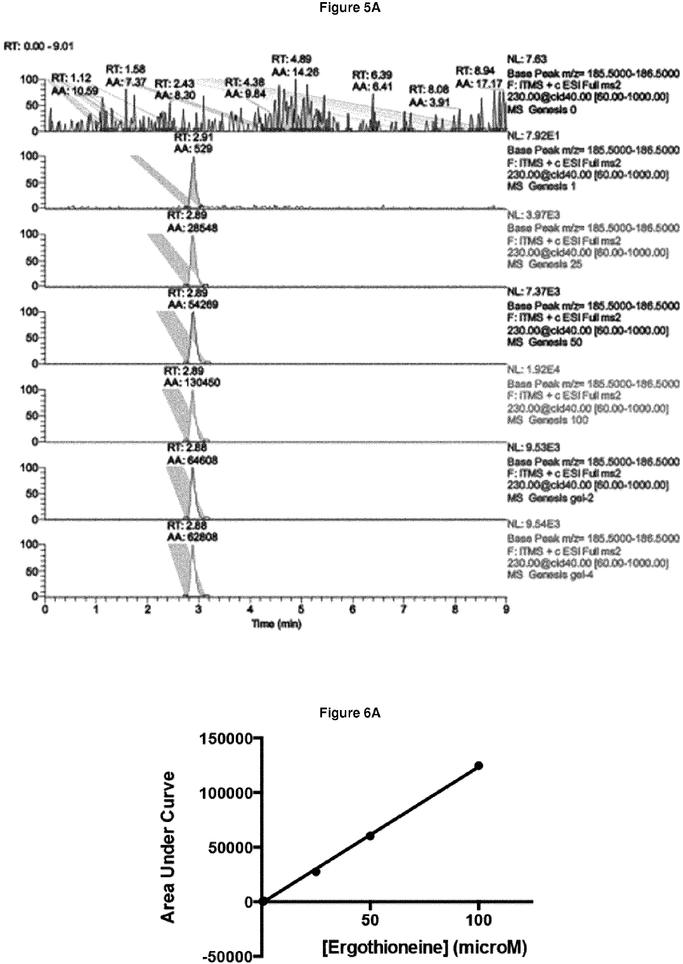

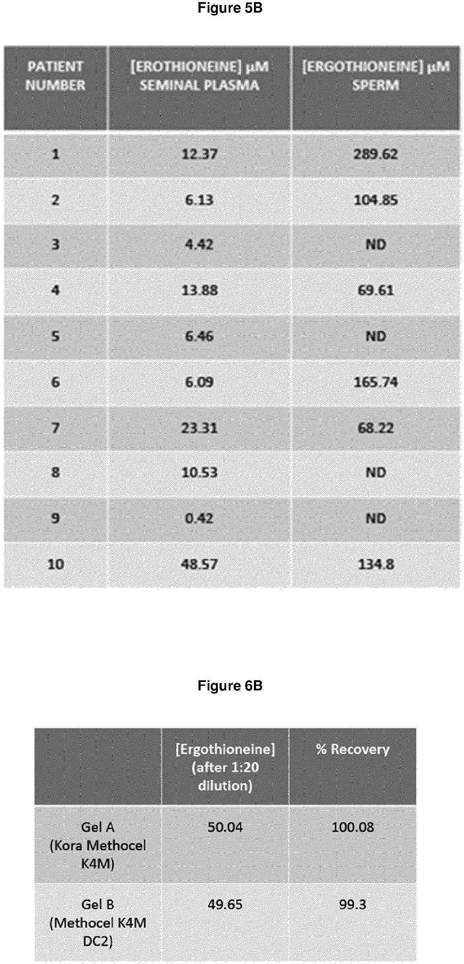

**Figure 5** illustrates ergothionine measurements in seminal plasma and sperm cells, wherein (A) to generate a standard curve, ergothioneine was prepared fresh in 50µl water and processed in the same manner to tissue samples as described, wherein data for each individual standard is shown; (B) protein precipitation with acetonitrile was used for bio-sample preparation prior to analysis, wherein sperm number of patient samples was counted using CASA and 10 million sperm removed and centrifuged at 500 xg for 2 min at 4°C, wherein the seminal plasma was removed, centrifuged again at 5000 xg for 2 min at 4°C and the supernatant removed and stored separately at -80°C, wherein the remaining sperm pellet was washed three times in PBS with centrifugation between each step at 500 xg, wherein pellets were stored at -80°C until used, wherein, on day of analysis, sperm pellets and seminal plasma were defrosted on ice; sperm pellets were made to 50µl with dH₂O and vortexed for 2 min; seminal plasma (50 µl) was transferred to a fresh tube, to 50µl of either sperm cells or seminal plasma 150 µl of acetonitrile was added, samples were vortexed for 2 min and then centrifuged at 13,000g for 30 min at 4°C, and after centrifugation, 30µl of supernatant was added to 170 µl of 0.1% Formic acid in water, and samples were vortexed briefly and analysed by LC-MS/MS;
**Figure 6** illustrates ergothionine is fully bio-available in the composition, wherein (A) ergothionine standards were prepared in water and analysed by LC-MS/MS to generate a standard curve; (B) the composition was prepared containing 0.23 mg/g w/w ergothionine (1 mM) and diluted 1:20 in water before being analysed by LC-MS/MS;

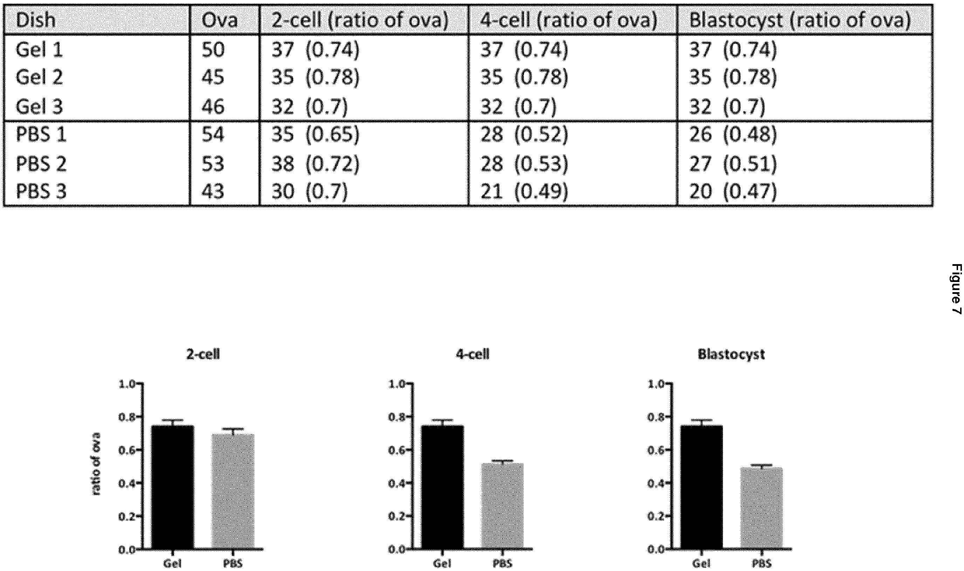

**Figure 7** illustrates the composition had no negative impact on sperm-egg fusion or early development, wherein mouse oocytes were placed with mouse sperm in fertilisation solution containing either (i) 10 % w/v composition in fertilisation medium or (ii) 10 % v/v PBS in fertilisation medium (control), wherein fertilisation was left to occur for 2.5 hours, presumptive zygotes were removed from fertilisation dishes and washed through 4 wash drops (no gel or PBS present) and counted, eggs were left to develop overnight and, the next morning, 2-cell embryos were removed and counted to fresh drop dishes and left to develop to blastocyst at which point they were again counted;

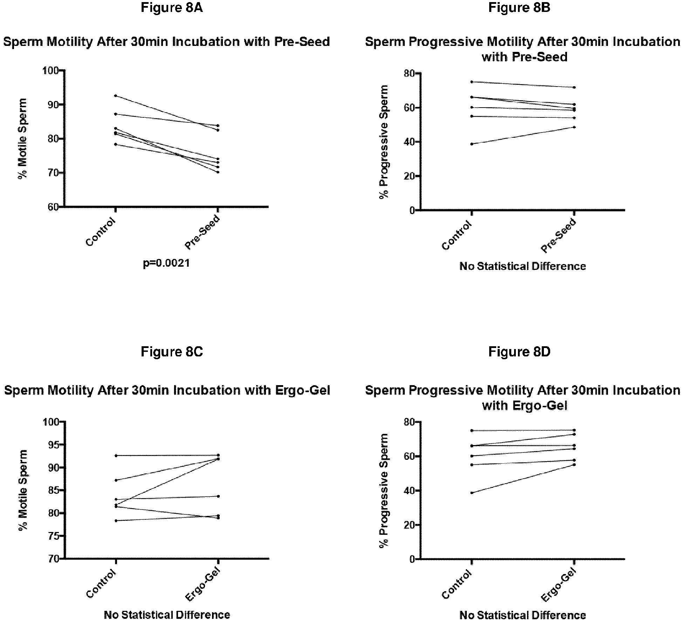

**Figure 8** illustrates a head-to-head comparison of the effect of a commercially available composition and the composition on sperm motility, wherein sperm were separated from seminal fluid by density gradient centrifugation, washed in sperm wash media and incubated in multipurpose handling medium in the presence of the commercially available composition (A,B) and the composition (C,D) for a period of 30 minutes, wherein sperm motility was analysed using CASA, wherein statistical analysis was performed using the Wilcoxon signed-rank test, wherein N=6 per group; and

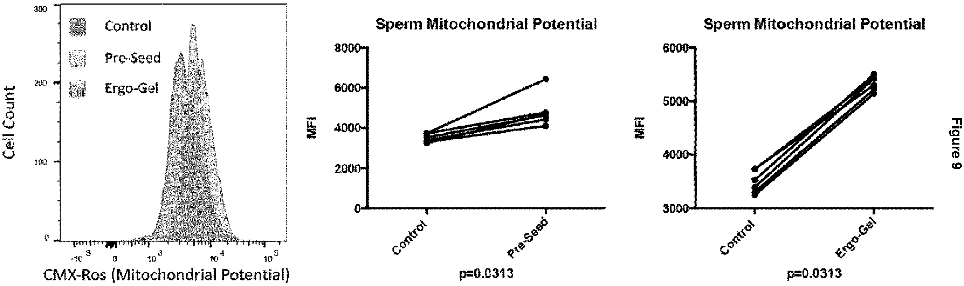

**Figure 9** illustrates a head-to-head comparison of the effect of the commercially available composition and the composition on sperm mitochondrial membrane potential, wherein sperm were separated from seminal fluid by density gradient centrifugation, washed in sperm wash media and incubated in multipurpose handling medium for 30 minutes in the presence of 10% v/v/ the commercially available composition or the composition, wherein sperm were stained with CMX-Ros and analysed by flow cytometry (A), wherein Mean Fluorescent Intensity (MFI) was determined for control and compared to MFI for the commercially available composition (B) and the composition (C), wherein statistical analysis was performed using the Wilcoxon signed-rank test, wherein N=6 per group.

### Materials & Methods

### Ethical approval

This study was carried out using semen samples from anonymous patients attending Merrion Fertility Clinic with permission from the donors and ethical approval from the School of Medicine, Research Ethics Committee, Trinity College Dublin and Merrion Fertility Clinic Ethics Committee. The research was carried out at Trinity Biomedical Sciences Institute, Trinity College Dublin.

### Study population and participants

Men attending the Merrion Fertility Clinic between August 2016 and February 2018 were invited to participate in this project and written consent was received from all participants. The inclusion criteria were samples from participants undergoing a fertility assessment. The exclusion criteria were samples from participants with azoospermia (no sperm) or oligospermia (low sperm count) with insufficient numbers to analyse or leukocytospermia (i.e. >1×10e6 white blood cells/mL).

### Sample collection and analysis

Semen samples were collected after 2-5 days sexual abstinence. Samples were collected in sterile plastic containers (Starstedt, 75-562-105) and allowed to liquefy for 30 minutes at 37°C. Semen analysis was performed within 1 h of ejaculation. All samples were subject to conventional analyses for semen volume (ml), sperm concentration (x 10e6 / ml), sperm morphology and motility, according to WHO recommendations (World Health Organisation (WHO) laboratory manual for the examination and processing of human semen (fifth edition, 2010)).

### CASA motility assessment

Sperm motility assessments were carried out by Computer Assisted Sperm Analysis (CASA) using the Hamilton Thorne software and a Zeiss microscope, following WHO and manufacturers' guidelines. Briefly, semen samples were resuspended using Pasteur pipettes and diluted 1 in 3 in sperm wash media (Irvine Scientific, 9983). A volume of 3 µl of diluted semen was added to the CASA microscope slide (Leja, 4 chamber slide, 20 microns, SC-20-01-04B), placed onto the preheated stage (37°C) and allowed to settle until no drift was apparent on screen. Motility assessment was evaluated by capturing at least 1000 sperm cell tracks per slide for unprocessed samples and 300 for processed samples. Total cell count (x 10e6/ml), percentage immotile (IM), non-progressively motile (NP) and progressively motile (PR) sperm and average path velocity (VAP) for M and PM sperm was recorded for each sample as defined by World Health Organisation (WHO) laboratory manual for the examination and processing of human semen (fifth edition, 2010).

### Sperm washing

Seminal plasma was removed by washing spermatozoa into 0.5 ml of sperm wash media (Irvine Scientific, 9983) by centrifugation at 1,500 x g for 1 minute at RT, repeated twice. The final sperm pellet was resuspended in 0.1 ml sperm wash media prior to downstream experiments.

### Density Gradient Centrifugation

Semen samples were purified by density gradient centrifugation (DGC). The Irvine Scientific separation media was used (Irvine Scientific, Upper Layer, 99257; Lower Layer, 99258). A volume of 1.5 ml of 40% lower layer separation media was added to a 15 ml falcon tube followed by 1.5 ml of 80% upper layer separation media. A minimum volume of 1 ml of the semen sample was loaded onto the gradient and centrifuged at 400 x g for 20 minutes at RT. Approximately 2.5 ml of the upper layer was gently removed using a sterile Pasteur pipette and the sperm pellet was washed twice with 2 ml sperm wash media, centrifuged at 380 x g for 5 minutes at RT. The washed pellet, containing highly motile purified sperm (>25 µm/sec), was resuspended in 0.5 ml of sperm wash media.

### Fixing human sperm samples

A total of 1 x 10e6 washed semen samples were fixed by re-suspending the washed sperm cell pellet in 500µl of 4% paraformaldehyde (Chem Cruz, 281692), pH 7.2 and incubating for 20-30 minutes at RT. Fixed spermatozoa were centrifuged at 2,500 x g for 1.5 minutes and washed twice in 500µl 1% NGS-PBS buffer.

### Quantitative assessment of oxidative DNA damage

Measurement of oxidative DNA damage was determined using anti-DNA/RNA [15A3] Damage-FITC antibody (Abcam, ab183393). Briefly, 1 x 10e6 sperm from seminal plasma were washed into 0.5 ml of sperm wash media followed by a two-step wash into 0.5 ml PBS with 1% FBS. Sperm were fixed using 4% PFA and washed twice into 0.5 ml 1% NGS-PBS buffer (NEB, 5424S). A total of 1 x 10e6 sperm were incubated with 2 µg/ml anti-DNA/RNA [15A3] Damage-FITC antibody in 0.1% Triton-X in 0.1% Sodium Citrate for 60 minutes at 37°C (Cambi et al., 2011). Samples were washed twice with 0.5 ml of PBS with 1% NGS prior to FC analysis. As a positive control, the anti-DNA/RNA [15A3] antibody was pre-incubated with 1,000-fold molar excess 8-hydroxy-2-guanosine for 30 minutes at RT prior to incubating with 1 x 10e6 spermatozoa for 1 hour at 37°C.

### Quantitative assessment of sperm cell health

Measurement of MMP and cell permeability were determined using MitoTracker (ThermoFisher, M7512) and DAPI (ThermoFisher, D1306), respectively. Briefly, 1 x 10e6 sperm from seminal plasma were washed into 0.5 ml of sperm wash media followed by a two-step wash into 0.5 ml PBS with 1% FBS. MMP was evaluated by incubation with 6.25 nM Mitotracker CMX Ros (Invitrogen, M7512) for 15 minutes at 37 °C. Cell permeability was evaluated by incubation with 35 ng/ml DAPI (Invitrogen, D1306) for 5 minutes at 37 °C in 0.1 ml PBS with 1% FBS, protected from light. Samples were washed twice with 0.5 ml of PBS with 1% FBS prior to FC analysis.

### Examples

### Example 1

### Ergothionine maintains sperm function over time

Ergothionine was evaluated-along with a range of compounds (not shown)-for its ability to maintain sperm health over a 48 hour time period, as determined by progressive motility. This was performed on a limited number of patient samples (n=5) and acted to identify effective lead compounds in addition to highlighting compounds with no discernible effect. From this initial screen, ergothionine was brought forward and tested for effects on sperm motility and average path velocity (VAP). The data were reviewed and graphed by Prof. Michael Parkinson-a highly experienced and recognised bio-statistician-and identified ergothionine as a compound with sperm protective properties (see Figure 1).

### Example 2

### Ergothionine protects sperm from accumulative DNA damage

ROS and DNA damage are the most significant contributors to male infertility. Therefore, optimally, a composition for use in the treatment of infertility would be capable of protecting against these deleterious effects on sperm health. Ergothionine has a recognised ability to shield DNA from the damaging effects of oxygen and lipid radicals. In measuring this parameter, a flow cytometric assay was developed that could quantify the amount of 8-hydroxyguanine (8-OH-G) in the DNA of individual sperm by the use of a specific fluorescently-labelled monoclonal antibody (see Figure 2).

8-OH-G is among the most abundant base adducts of DNA caused by ROS and is regarded as a suitable biomarker for oxidative DNA damage. Incubation of sperm with ergothionine (0.1 mM) demonstrated the ability to protect against 8-OH-G formation on sperm DNA over a 48 hour time period (see Figure 3a).

The effect of ergothionine was found to be dose-dependant since higher concentrations of ergothionine, at 1 mM, generated an even greater cytoprotective effect showing statistical improvement over the 0.1 mM concentration (see Figure 3b).

The data highlight two key points: ergothionine is effective at a concentration of 0.1 mM and therefore the final composition formulation can be prepared at 1 mM (it is expected that the sperm will come in contact with a minimum 10% concentration *in vivo*) and at the 1 mM concentration ergothionine is effective and non-toxic, with no negative effect on sperm motility and progression over a 48 hour time period.

### Example 3

### Ergothionine is an effective cytoprotective agent for sperm motility and progression

Having demonstrated that ergothionine can protect against accumulative DNA damage, studies were performed to determine the effect on sperm motility at 1 mM over a 24 hour (A-C) and 48 hour (D-F) time period and at a concentration of 0.1 mM over a 24 hour (G-I) and 48 hour (J-L) (see Figure 4).

Data showed that ergothionine was effective at maintaining the percentage of motile and progressive sperm in addition to the speed of the movement (VAP), with p values ≤ 0.002 for all parameters at 1 mM and p values ≤ 0.04 for all parameters at 0.1 mM.

### Example 4

### Ergothionine is present in seminal plasma and sperm

Ergothionine is a naturally occurring micronutrient with a specific transporter (OCTN1) that has been shown to be present on sperm cells. In addition, boar and stallion seminal plasma are known to contain high micromolar (-0.3-0.8 mM) concentrations of ergothionine. However, ergothionine levels have not previously been determined in human sperm cells. Our studies, using mass spectrometric analysis demonstrate that human sperm cells and seminal plasma contain significant although variable levels of ergothionine in the range of 0.42-49 µM and 0-290 µM, respectively (see Figure 5).

The data have two important consequences:
(i) the presence of ergothionine within human sperm suggested an existing natural protective function and argues against any potential negative effects on fertility and;
(ii) the inclusion of ergothionine in a gel formulation would allow sperm to accumulate the compound intracellularly to illicit its cytoprotective effect until fusion with the ovum.

### Example 5

### Formulating a sperm and egg friendly composition

A composition with intended use in conception must be capable of satisfying a number of important criteria:
(i) as a condition of its use in the treatment of infertility it should have an appropriate consistency and extrudibilty. However, the viscosity should not adversely affect sperm motility or the mucosal environment of the female reproductive tract;
(ii) the composition should provide the correct osmolarity (between 260-360 mOsm/kg) in a similar range to that of the seminal plasma. Available pre-clinical and clinical data support that hyperosmolal vaginal products may be related to detrimental effects on sperm motility, viability and chromatin quality; and
(iii) the pH should favour sperm survival, ostensibly in a similar range to that of the seminal plasma (pH of 7-8.5). The optimal pH for sperm (∼pH 7.0-7.2) differs from that of the normal vaginal pH in the healthy adult (pH 3.5-4.5), the disruption of which can induce bacterial vaginosis resulting from the overgrowth of atypical bacteria within the vagina and this condition has been linked to early miscarriage. Furthermore, sperm require an acidic pH immediately post ejaculation to fuse with prostasomes (exosomes secreted by the prostate), which confer a number of complement inhibiting proteins onto the sperm surface so as to evade subsequent immune attack in utero as well as a number of lipids that stabilize the outer membrane of the sperm and prevent early acrosome reaction. Therefore, the composition should be designed to have a low buffering capacity that is insufficient to significantly offset the normal pH.

With these parameters in mind a composition was prepared with the following composition 1-1.4% HPMC, 0.3-0.5% Carbomer, 5 mM NaCl, 5 mM NaPO₄, 0.23 mg/g w/w ergothionine (1 mM) and has the following chemical and physical characteristics pH 7.0-7.2, osmolality 320-360 mOsm/kg, and viscosity 6-20 Pa.s.

To be effective as a cytoprotective agent in the context of the composition for use in the treatment of infertility it is necessary that the ergothionine can be recovered to high levels. To evaluate this, liquid chromatography-tandem mass spectrometry was employed and the percentage recovery calculated based on a standard curve (see Figure 6). It is apparent from the data that the ergothionine is completely recoverable from the composition and therefore highly likely to elicit the expected cytoprotective effects *in vivo.*

### Example 6

### Ergothionine has no negative effect on egg fertilisation and early development

To function as a composition for use in the treatment of infertility it is essential that the formulation does not hamper vital sperm mechanisms such as prostasome-sperm fusion, motility, capacitation, acrosome reaction and have no impact on sperm engagement with the egg or early embryo development. To evaluate this, *in vitro* fertilisation studies were performed in the presence of the composition at a concentration of 10% and subsequent embryo development examined to blastocyst stage (see Figure 7). Notably, there was no negative effect on any of the parameters of egg fertilisation, morula or blastocyst development.

### Example 7

Head-to-head comparison of the composition against a commercially available composition (Pre-Seed^{™})^{.}

The percentage motile sperm and progressive motility are commonly used parameters for evaluating fertility in males. A direct comparison was made between the effects that the commercially available composition and the composition have on these parameters at a 10% concentration over a 30 minute incubation period (n=6); note that sperm would be expected to come in contact with lubricant for approximately 20 minutes in the *in vivo* situation (see Figure 8).

It is clear that the commercially available composition is having a negative effect on the percentage of motile sperm over the incubation period and despite only comprising six samples the effect is statistically significant indicating that the deleterious effect is quite marked. By contrast, the composition slows no deleterious effects.

A further parameter that can be used to report on sperm health is mitochondrial activity. Mitochondrial activity can be reported on through evaluation of membrane potential. A comparison of the composition against the commercially available composition was made by incubating sperm for 30 minutes in a 10% concentration in multipurpose handling medium.

Both formulations had the ability to preserve mitochondrial membrane potential. Therefore it is concluded that at both the cellular and subcellular level the composition performs to maintain sperm health over time.

The composition of the invention is sperm and egg friendly and has no negative effects on fertilisation or early embryo development. The data in Example 6 show that the sperm exposed to ergothionine are more effective that those exposed to PBS and also that early embryo development (to blastocyst stage) is unaffected. The antioxidant in the composition of the invention has demonstrable cytoprotective effects against oxidative DNA damage at concentrations of 0.1 and 1 mM in line with the concentrations found in the composition (1 mM) and the concentrations that the sperm cells are likely to experience *in vivo* (∼0.1 mM). Importantly, our data show that human seminal plasma and sperm itself contain significant, although variable amounts of the antioxidant, in line with the fact that the transporter for the antioxidant OCTN1 is found on the vast majority of cells including sperm cells.

Studies have demonstrated that high doses of the antioxidant are safe for consumption with no adverse effects observed when given to rats at a concentration of 2 g/kg. In addition, it has previously been shown that the antioxidant is safe for embryo development at 0.1 mM and displays no negative impact on fertility in rats fed at a concentration of 0.9% of diet for 13 weeks. Although high concentrations of the antioxidant (>0.5 mM) were found to negatively affect bovine embryo development, concentrations up to 0.1 mM proved beneficial (Halliwell, B., Cheah, I.K. and Tang, R.M., 2018. Ergothioneine-a diet-derived antioxidant with therapeutic potential. Febs Letters, 592(20), pp.3357-3366). Studies on sheep embryos indicated that concentrations up to 10 mM were beneficial for *in vitro* maturation of sheep oocytes.

The antioxidant in the composition of the invention is a colorless and odourless water soluble small-molecule antioxidant with the ability to neutralize free radicals in a process called radical scavenging. Numerous antioxidant and cytoprotective effects have been demonstrated including the ability to neutralise free radicals, protect from radiological and UV damage and to act an anti-inflammatory agent, protect against ischemia-reperfusion injury and acute lung injury, among other positive effects. *In vitro* studies have shown its ability to react with hydroxyl radicals (•OH), hypochlorous acid (HOCI) and peroxynitrite (ONOO-), to deactivate singlet oxygen and also to act as a metal chelator thus preventing the Fenton reaction. Notably, it is believed that the antioxidant function of the antioxidant accounts for its conserved accumulation by humans and other metazoans (organisms containing a gut) i.e. the recovery and uptake of this amino acid from fungi is desirable for the protective activity it bestows.

The composition of the invention is formulated to provide physical parameters suitable for maintaining sperm function and compatible for use in the female reproductive tract (appropriate viscosity, buffering capacity and osmolality). *In vitro* fertilisation studies using mice gametes demonstrate the composition does not impair fertilisation and has no negative impact on early embryo development. The data described in this disclosure demonstrate that the composition is an appropriate product for use by couples attempting to conceive.

## Claims

1. A composition comprising an antioxidant for use in the treatment of infertility.

2. A composition for use according to Claim 1, wherein the antioxidant is ergothionine.

3. A composition for use according to Claim 1 or 2, wherein the composition comprises 0.1 - 20.0mM antioxidant.

4. A composition for use according to any one of Claims 1-3, wherein the composition further comprises an excipient.

5. A composition for use according to Claim 4, wherein the excipient is hydroxypropyl methylcellulose (HPMC).

6. A composition for use according to Claim 4 or 5, wherein the composition comprises 0.8 - 1.6 wt% excipient.

7. A composition for use according to any one of Claims 1-6, wherein the composition further comprises a thickening agent.

8. A composition for use according to Claim 7, wherein the thickening agent is a polyacrylic acid.

9. A composition for use according to Claim 7 or 8, wherein the composition comprises 0.1 - 1.0 wt% thickening agent.

10. A composition for use according to any one of Claims 1-9, wherein the composition further comprises at least one salt, the or each salt independently selected from NaCl and NaPO₄.

11. A composition for use according to Claim 10, wherein the composition comprises 2.5 - 7.5 mM salt.

12. A composition for use according to any one of Claims 1-11, wherein the composition has an osmolarity of 260 - 360 mOsm/kg.

13. A composition for use according to any one of Claims 1-12, wherein the composition has a pH of 7.0 - 8.5.

14. A composition for use according to any one of Claims 1-13, wherein the composition has a viscosity of less than 2500 Pa.s at 20°C.

15. A composition for use according to any one of Claims 1-14, wherein the composition is for use in increasing sperm motility or sperm velocity.
